# EUROPÄISCHE PATENTSCHRIFT

(11) **EP 3 856 071 B1**
(45) Veröffentlichungstag und Bekanntmachung des Hinweises auf die Patenterteilung: **05.10.2022**
(21) Anmeldenummer: 19778951.4
(22) Anmeldetag: 25.09.2019
(51) Int. Cl.: A61C 1/10, A61C 1/12, A61C 1/14, A61B 17/16

(54) **DENTALES ODER CHIRURGISCHES HANDSTÜCK MIT EINEM RFID-TRANSPONDER**
DENTAL OR SURGICAL HANDPIECE HAVING AN RFID TRANSPONDER
PIÈCE À MAIN DENTAIRE OU CHIRURGICALE DOTÉE D'UN TRANSPONDEUR RFID

(30) Priorität: 26.09.2018 DE 102018123694
(43) Veröffentlichungstag der Anmeldung: 04.08.2021
(73) Patentinhaber: KaVo Dental GmbH, 88400 Biberach (DE)
(72) Erfinder: SAUTER, Johannes, 88416 Mittelbuch (DE); STEMPFLE, Johann, 89284 Pfaffenhofen (DE)
(74) Vertreter: Thun, Clemens
(86) Internationale Anmeldenummer: PCT/EP2019/075785
(87) Internationale Veröffentlichungsnummer: WO 2020/064789

(56) Entgegenhaltungen:
- EP-A1- 2 581 061
- EP-A1- 3 067 008
- EP-A2- 2 233 103
- DE-A1-102010 011 630

## Beschreibung

Die Erfindung betrifft ein Kopfstück für ein dentales oder chirurgisches Handstück mit einem RFID-Transponder (RFID: radio-frequency identification).

In der medizinischen Praxis wird üblicherweise die Verwendung von Hilfsmitteln und Geräten nachweisbar gehandhabt. Dies betrifft auch die Aufbereitung und den Hygienezustand (z. B. Sterilität) der Hilfsmittel bzw. Geräte. Überwiegend dienen hierzu manuelle Einträge oder Einträge in einem Praxismanagement Programm. Es sind auch Systeme im Einsatz, bei welchen ein am jeweiligen Instrument angebrachter Datamatrix-Code mit einem Scanner erfasst wird, so dass eine entsprechende manuelle Eingabe nicht erforderlich ist und damit die Gefahr eines Übertragungsfehlers vermieden werden kann.

Bei den aktuell verwendeten Methoden muss zur Registrierung der Code bzw. die Kennung eingelesen oder eingegeben werden. Eine Informationsübertragung zum betreffenden Instrument, beispielsweise mit Bezug auf dessen Hygienezustand oder die Anzahl der Benutzungen ist hierbei nicht möglich.

Aus der EP 3 067 008 A1 ist ein dentales Handstück mit einem, im Griffstück integrierten RFID-Transponder bekannt. Zur Herstellung dieses Handstücks ist es erforderlich, ein Stück des aus Metall bestehenden Griffstücks auszusparen, den RFID-Transponder dort anzuordnen und anschließend die Aussparung mit einem Kunststoffmaterial zu bedecken, damit in funktechnisch ungünstigen Umgebungen (z. B. Metall) Daten ausgelesen werden können. Die Herstellung ist somit mit einem erheblichen Aufwand verbunden.

Außerdem lässt sich das aus der EP 3 067 008 A1 bekannte Handstück schwerlich durch ein "Nachrüsten" eines Handstücks ohne RFID-Transponder gewinnen.

Ferner zeigt die DE 10 2010 011 630 A1 ein dentales oder chirurgisches Handstück, welches eine längliche Griffhülse sowie ein am vorderen Ende der Griffhülse angeordnetes Kopfstück aufweist, welches dazu dient, ein zahnmedizinisches Werkzeug lösbar aufzunehmen. Ein in dem Kopfstück ausgebildeter Einspannmechanismus zum lösbaren Halten des Werkzeugs weist einen Druckknopf auf, der in seinem Bodenbereich mit einer Aussparung versehen ist. Diese dient dazu, das Eingreifen einer Feder zu ermöglichen, wodurch die Bauhöhe des Kopfbereichs reduziert werden kann.

Die Schrift EP 2 581 061 A1 zeigt einen Kopplungsmechanismus zwischen einem medizinischen Instrument und einer Antriebseinheit, wobei die Kopplung derart erfolgt, dass nach Kopplung der beiden Komponenten Daten von der einen Komponenten mittels einer Übertragungseinheit auf die andere Komponente übertragbar sind.

Der Erfindung liegt die Aufgabe zugrunde, ein entsprechendes verbessertes Kopfstück für ein Handstück anzugeben. Insbesondere soll das Kopfstück Vorteile mit Bezug auf seine Herstellungsmöglichkeiten und seine elektrischen Eigenschaften bieten können.

Diese Aufgabe wird gemäß der Erfindung mit den in den unabhängigen Ansprüchen genannten Gegenständen gelöst. Besondere Ausführungsarten der Erfindung sind in den abhängigen Ansprüchen angegeben.

Gemäß der Erfindung ist ein Kopfstück für ein dentales oder chirurgisches Handstück vorgesehen, wobei das Kopfstück dazu dient, ein zahnmedizinisches Werkzeug lösbar aufzunehmen und wobei in dem Kopfstück ein Einspannmechanismus zum lösbaren Halten des Werkzeugs ausgebildet ist, der einen Druckknopf zum Betätigen des Einspannmechanismusses aufweist. Weiterhin weist das Handstück einen, zum Identifizieren des Handstücks vorgesehenen RFID-Transponder auf. Dabei besteht der Druckknopf aus einem wärmeisolierenden, elektrisch nichtleitendem Kunststoff- oder Keramikmaterial, in welchem eine tellerförmige Vertiefung ausgebildet ist, in welcher der RFID-Transponder angeordnet ist.

Der Druckknopf hat eine flache runde Form, so dass sich der RFID-Transponder aufgrund der ebenfalls runden flachen Form seiner Spule sehr gut in den Druckknopf integrieren lässt. Dennoch ist der RFID-Transponder gut vor mechanischen Einflüssen geschützt angeordnet.

Da der Druckknopf aus einem elektrisch nichtleitendem Material besteht, lassen sich besonders gute elektrische Umgebungsbedingungen für den RFID-Transponder erzielen; insbesondere lässt sich so vorteilhaft ein "Luftspalt" für den RFID-Transponder realisieren bzw. ein geeigneter Abstand zu metallenen Bauteilen des Handstücks. Dabei befindet sich der Druckknopf an einer exponierten Stelle des Handstücks, wodurch weiterhin besonders gute Empfangsbedingungen ermöglicht sind.

Dadurch, dass der Druckknopf aus einem wärmeisolierenden Material besteht, ist eine thermische Isolierung gebildet. Auf diese Weise lässt sich die Gefahr vermindern, dass sich der Druckknopf unerwünscht erwärmt - beispielsweise bedingt durch eine Drehung des Werkzeugs - und so zu einer Verbrennung eines Nutzers des Handstücks oder eines Patienten führt.

Außerdem handelt es sich bei dem Druckknopf um ein Bauteil, das unabhängig von einem Griffstück bzw. einer Griffhülse eines dentalen oder chirurgischen Handstücks gestaltet ist. Daher ist es möglich, ein entsprechendes Handstück, das einen Druckknopf ohne RFID-Transponder aufweist, lediglich durch Austausch des Druckknopfes gegen einen anmeldungsgemäßen Druckknopf mit einem RFID-Transponder "nachzurüsten".

Vorzugsweise ist der in der Vertiefung angeordnete RFID-Transponder mit einer Vergussmasse vergossen. Dies ist herstellungstechnisch vorteilhaft und ermöglicht dabei besonders gute elektrische Umgebungsverhältnisse für den RFID-Transponder.

Vorzugsweise weist der RFID-Transponder einen Abstand zu einer Bodenfläche der tellerförmigen Vertiefung auf, wobei der Bereich zwischen dem RFID-Transponder und der Bodenfläche durch die Vergussmasse gefüllt ist. So lässt sich erzielen, dass zwischen der Antenne des RFID-Transponders und einem metallenen Untergrund "Luft" ist, so dass die Feldlinien die Antenne gut durchfluten können.

Vorzugsweise bildet die, die tellerförmige Vertiefung ausfüllende und den RFID-Transponder umgebende Vergussmasse einen gegenüber dem umgebenden Oberflächenbereich des Druckknopfs erhöhten Bereich, wobei die Vertiefung und der erhöhte Bereich vorzugsweise eine etwa kreisförmige Außenkontur aufweisen. Dies ist vorteilhaft mit Bezug auf die elektrischen Umgebungsbedingungen der Antenne des RFID-Transponders. Hierzu ist weiterhin vorzugsweise der maximale Überstand der Vergussmasse gegenüber dem umgebenden Oberflächenbereich des Druckknopfs kleiner als 2 mm, vorzugsweise beträgt er 1 mm bis 2 mm.

Weiterhin vorzugsweise setzt dabei ergonomisch vorteilhaft die überstehende Vergussmasse den umgebenden Oberflächenbereich des Druckknopfs im Wesentlichen stetig fort.

Vorzugsweise ist der Druckknopf an einer dem Werkzeug gegenüberliegenden Oberseite des Kopfstücks angeordnet, wobei die tellerförmige Vertiefung vorzugsweise im Wesentlichen mittig an dem Druckknopf ausgebildet ist. So lässt sich der Druckknopf besonders geeignet zum Betätigen des Einspannmechanismus gestalten.

Bei dem Kunststoff handelt es sich vorzugsweise um Polyetheretherketon (PEEK); dieses Material ist wiederholt sterilisierbar. Die Vergussmasse weist vorzugsweise Harz und/oder Silikon auf.

Gemäß einem weiteren Aspekt der Erfindung ist ein dentales oder chirurgisches Handstück mit einer länglichen Griffhülse vorgesehen, wobei am vorderen Ende der Griffhülse ein Kopfstück entsprechend der vorherigen erfindungsgemäßen Ausführung angeordnet ist.

Die Griffhülse und das Kopfstück bestehen anwendungsfreundlich vorzugsweise aus einem Metall. Die Griffhülse ist vorzugsweise handhabungsfreundlich abgewinkelt ausgeführt.

Besonders eignet sich das Handstück, wenn dieses in der Griffhülse einen Antriebsmotor oder eine zur Kopplung mit einem externen Antriebsmotor vorgesehene Welle aufweist, wobei ferner in der Griffhülse ein Antriebsstrang gelagert ist zur Übermittlung einer Rotationsbewegung auf das an dem Kopfstück gehaltene Werkzeug. Der Einspannmechanismus weist vorzugsweise eine, einen Schaft des Werkzeugs haltende Spannzange auf.

Die Erfindung wird im Folgenden anhand eines Ausführungsbeispiels und mit Bezug auf die Zeichnungen näher erläutert. Es zeigen:
- Fig. 1: eine Skizze eines anmeldungsgemäßen Handstücks,
- Fig. 2: eine Querschnitt-Skizze durch das Kopfstück des Handstücks und
- Fig. 3: eine vergrößerte Darstellung aus Fig. 2 um den Bereich des Druckknopfes.

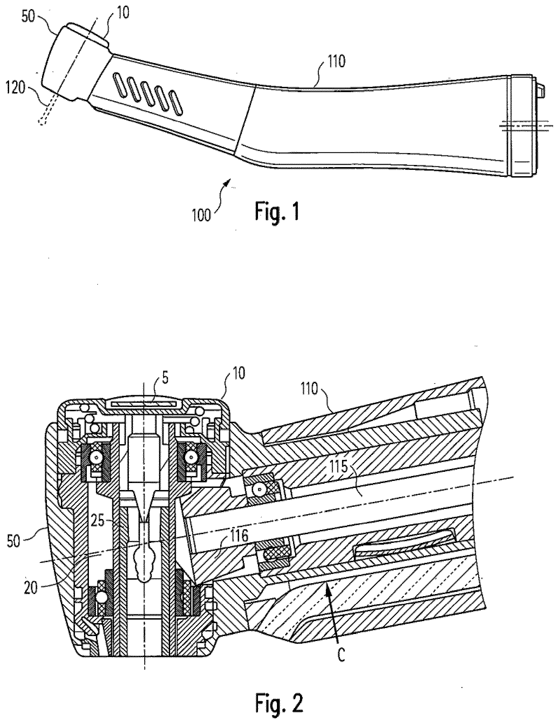

Fig. 1 zeigt eine Skizze einer seitlichen Ansicht eines anmeldungsgemäßen dentalen oder chirurgischen Handstücks 100. Das Handstück 100 weist eine längliche Griffhülse 110 auf. Vorzugsweise ist am vorderen Ende der Griffhülse 110 ein Kopfstück 50 angeordnet, das dazu dient, ein zahnmedizinisches Werkzeug 120 lösbar aufzunehmen. Die Griffhülse 110 und das Kopfstück 50 bestehen vorzugsweise aus Metall. Wie in Fig. 1 beispielhaft skizziert, ist die Griffhülse 110 vorzugsweise ergonomisch vorteilhaft abgewinkelt ausgeführt.

Bei dem Handstück 100 kann es sich insbesondere um ein so genanntes Winkelstück oder um eine so genannte Turbine handeln.

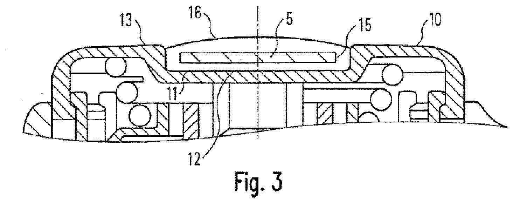

Fig. 2 zeigt eine Querschnitt-Skizze durch das Kopfstück 50. In dem Kopfstück 50 ist ein Einspannmechanismus 20 zum lösbaren Halten des Werkzeugs 120 ausgebildet, der einen Druckknopf 10 zum Betätigen des Einspannmechanismus 20 aufweist. Fig. 3 zeigt den Bereich um den Druckknopf 10 näher.

Weiterhin umfasst das Handstück 100 einen, zum Identifizieren des Handstücks 100 vorgesehenen RFID-Transponder 5.

Der Druckknopf 10 besteht aus einem wärmeisolierenden, elektrisch nichtleitenden Kunststoff- oder Keramikmaterial. Bei dem Kunststoffmaterial kann es sich insbesondere um PEEK handeln. Wenn der Druckknopf 10 aus einem Keramikmaterial besteht, kann eine Information, beispielsweise ein Logo oder dergleichen dauerhaft angebracht werden, zum Beispiel mittels CO2-Laser.

In bzw.an dem Druckknopf 10 ist eine tellerförmige Vertiefung 11 ausgebildet, in welcher der RFID-Transponder 5 angeordnet ist. Dabei ist der RFID-Transponder 5 mit einer Vergussmasse 15, die vorzugsweise Harz und/oder Silikon aufweist, vergossen.

Wie im gezeigten Beispiel der Fall, weist der RFID-Transponder 5 dabei einen Abstand zu einer Bodenfläche 12 der tellerförmigen Vertiefung 11 auf, wobei der Bereich zwischen dem RFID-Transponder 5 und der Bodenfläche 12 durch die Vergussmasse 15 gefüllt ist.

Wenn im Allgemeinen ein RFID-Transponder zu nahe an einem metallenen Gegenstand platziert ist, laufen die Feldlinien in das Metall und sind nicht geschlossen; in diesem Fall kann keine Kommunikation zwischen dem RFID-Transponder 5 und einer entsprechenden Empfangseinheit stattfinden. Durch den hier beschriebenen Aufbau lässt sich anmeldungsgemäß erzielen, dass zwischen der Antenne des RFID-Transponders 5 und einem metallenen Untergrund noch "Luft" ist, so dass die Feldlinien die Antenne gut durchfluten können und um den RFID-Transponder 5 in geschlossener Form verlaufen. Auf diese Weise lassen sich vorteilhafte elektrische Umgebungsbedingungen für den RFID-Transponder 5 bewirken.

Über einen im RFID-Transponder 5 enthaltenen elektrischen Speicher kann beispielsweise eine eindeutige Identifikation des Handstücks 100 durchgeführt werden. Eine Information über einen Hygienezustand des Handstücks 100, eine Nutzungsdauer, eine Nutzungshäufigkeit u. s. w. kann in einem Schreib-Lese-Speicher des RFID-Transponders 5 abgespeichert werden oder auch in einem Speicher eines zentralen Management-Systems.

Auf diese Weise lässt sich bewirken, dass dem Handstück 100 Informationen über seinen Zustand sozusagen "anhaften"; diese können bei einer Nutzung oder Aufbereitung des Handstücks 100 aktualisiert werden und gegebenenfalls auch an das zentrale Management-System übertragen werden.

Die Vergussmasse 15, die die tellerförmige Vertiefung 11 ausfüllt und den RFID-Transponder 5 umgibt, bildet, wie aus Fig. 3 ersichtlich, vorzugsweise einen, gegenüber dem umgebenden Oberflächenbereich 13 des Druckknopfs 10 erhöhten Bereich 16. Dabei weisen die Vertiefung 11 und der erhöhte Bereich 16 vorzugsweise eine etwa kreisförmige Außenkontur auf. Grundsätzlich kann der RFID-Transponder 5 aus dem Druckknopf 10 mehr oder weniger hervorstehend angeordnet sein. Auf diese Weise lässt sich die Größe des Abstands zur Bodenfläche 12, also die "Spaltbreite" einstellen. Allerdings sollte der RFID-Transponder 5 nicht zu weit herausstehen, da in diesem Fall das Kopfstück 50 mit dem Druckknopf 10 und dem RFID-Transponder 5 insgesamt eine Größe erreicht, die grundsätzlich unerwünscht ist.

Daher ist der maximale Überstand der Vergussmasse 15 gegenüber dem umgebenden Oberflächenbereich 13 des Druckknopfs 10 vorzugsweise kleiner als 2 mm, besonders bevorzugt beträgt er 1 mm bis 2 mm.

Weiterhin vorzugsweise ist die Gestaltung derart, dass die überstehende Vergussmasse 15 den umgebenden Oberflächenbereich 13 des Druckknopfs 10 im Wesentlichen stetig fortsetzt.

Vorzugsweise weist das Handstück 100 in der Griffhülse 110 einen Antriebsmotor oder eine zur Kopplung mit einem externen Antriebsmotor vorgesehene Welle 115 auf, wobei ferner in der Griffhülse 110 ein Antriebsstrang 116 zur Übermittlung einer Rotationsbewegung auf das an dem Kopfstück 50 gehaltene Werkzeug 120 gelagert ist. Der Einspannmechanismus 20 weist vorzugsweise eine Spannzange 25 auf, die dazu gestaltet ist, einen Schaft des Werkzeugs 120 zu halten.

Wie aus Fig. 2 hervorgeht, ist im gezeigten Beispiel der Druckknopf 10 an einer, dem Werkzeug 120 gegenüberliegenden Oberseite des Kopfstücks 50 angeordnet, wobei die tellerförmige Vertiefung 11 vorzugsweise im Wesentlichen mittig an dem Druckknopf 10 ausgebildet ist. Insbesondere kann der Druckknopf 10 zur Betätigung der Spannzange 25 ausgebildet sein.

Folgende Merkmale tragen zur Verbesserung der Lese-, Schreib- bzw. Abstandbedingungen bei:
- Die Antenne des RFID-Transponders 5 weist einen kleinen Abstand (Luftspalt) zu einer metallenen Umgebung auf.
- Die Antenne des RFID-Transponders 5 ragt aus der metallenen Umgebung heraus.
- Die Antenne des RFID-Transponders 5 befindet sich auf bzw. in dem Druckknopf 10, der aus einem elektrisch nichtleitenden und nicht magnetischem Werkstoff bestehen kann.
- Der Druckknopf 10 besteht aus Kunststoff oder Keramik.
- Der RFID-Transponder 5 ist von Kunststoff bzw. Keramik ummantelt.

Zur Herstellung kann vorgesehen sein, dass ein Tropfen der Vergussmasse 15 auf die Bodenfläche 12 der Vertiefung 11 gegeben wird, so dass der Tropfen den Spalt bildet, Anschließend wird der RFID-Transponder 5 mit weiterer Vergussmasse darauf vergossen, wobei der Tropfen noch nicht getrocknet ist und somit eine einheitliche Vergussmasse gebildet wird.

## Patentansprüche

1. Kopfstück (50) für ein dentales oder chirurgisches Handstück (100),
wobei das Kopfstück (50) dazu dient, ein zahnmedizinisches Werkzeug (120) lösbar aufzunehmen,
wobei in dem Kopfstück (50) ein Einspannmechanismus (20) zum lösbaren Halten des Werkzeugs (120) ausgebildet ist, der einen Druckknopf (10) zum Betätigen des Einspannmechanismusses (20) aufweist,
**dadurch gekennzeichnet,**
**dass** der Druckknopf (10) aus einem wärmeisolierenden, elektrisch nichtleitenden Kunststoff- oder Keramikmaterial besteht, in welchem eine tellerförmige Vertiefung (11) ausgebildet ist, in der ein zum Identifizieren des Handstücks (100) vorgesehener RFID-Transponder (5) angeordnet ist.

2. Kopfstück für ein dentales oder chirurgisches Handstück nach Anspruch 1,
**dadurch gekennzeichnet,**
**dass** der in der Vertiefung (11) angeordnete RFID-Transponder (5) mit einer Vergussmasse (15) vergossen ist.

3. Kopfstück für ein dentales oder chirurgisches Handstück nach Anspruch 2,
**dadurch gekennzeichnet,**
**dass** der RFID-Transponder (5) einen Abstand zu einer Bodenfläche (12) der tellerförmigen Vertiefung (11) aufweist, wobei der Bereich zwischen dem RFID-Transponder (5) und der Bodenfläche (12) durch die Vergussmasse (15) gefüllt ist.

4. Kopfstück für ein dentales oder chirurgisches Handstück nach Anspruch 2 oder 3,
**dadurch gekennzeichnet,**
**dass** die die tellerförmige Vertiefung (11) ausfüllende und den RFID-Transponder (5) umgebende Vergussmasse (15) einen gegenüber dem umgebenden Oberflächenbereich (13) des Druckknopfs (10) erhöhten Bereich (16) bildet, wobei die Vertiefung (11) und der erhöhte Bereich (16) vorzugsweise eine etwa kreisförmige Außenkontur aufweisen.

5. Kopfstück für ein dentales oder chirurgisches Handstück nach Anspruch 4,
**dadurch gekennzeichnet,**
**dass** der maximale Überstand der Vergussmasse (15) gegenüber dem umgebenden Oberflächenbereich (13) des Druckknopfs (10) kleiner als 2mm, vorzugsweise 1mm bis 2mm beträgt.

6. Kopfstück für ein dentales oder chirurgisches Handstück nach Anspruch 5,
**dadurch gekennzeichnet,**
**dass** die überstehende Vergussmasse (15) den umgebenden Oberflächenbereich (13) des Druckknopfs (10) im Wesentlichen stetig fortsetzt.

7. Kopfstück für ein dentales oder chirurgisches Handstück nach einem der vorherigen Ansprüche,
**dadurch gekennzeichnet,**
**dass** der Druckknopf (10) an einer dem Werkzeug (120) gegenüberliegenden Oberseite des Kopfstücks (50) angeordnet ist, wobei die tellerförmige Vertiefung (11) vorzugsweise im Wesentlichen mittig an dem Druckknopf (10) ausgebildet ist.

8. Kopfstück für ein dentales oder chirurgisches Handstück nach einem der vorherigen Ansprüche,
**dadurch gekennzeichnet,**
**dass** es sich bei dem Kunststoff um Polyetheretherketon (PEEK) handelt.

9. Kopfstück für ein dentales oder chirurgisches Handstück nach einem der vorherigen Ansprüche,
**dadurch gekennzeichnet,**
**dass** die Vergussmasse (15) Harz und/oder Silikon aufweist.

10. Kopfstück für ein dentales oder chirurgisches Handstück nach einem der vorherigen Ansprüche,
**dadurch gekennzeichnet,**
**dass** der Einspannmechanismus (20) eine einen Schaft des Werkzeugs (120) haltende Spannzange (25) aufweist.

11. Dentales oder chirurgisches Handstück mit
einer länglichen Griffhülse (110) und
einem am vorderen Ende der Griffhülse (110) angeordneten Kopfstück (50) nach einem der vorherigen Ansprüche.

12. Dentales oder chirurgisches Handstück nach Anspruch 11,
**dadurch gekennzeichnet,**
**dass** die Griffhülse (110) und das Kopfstück (50) aus Metall bestehen.

13. Dentales oder chirurgisches Handstück nach Anspruch 11 oder 12,
**dadurch gekennzeichnet,**
**dass** die Griffhülse (110) abgewinkelt ausgeführt ist.

14. Dentales oder chirurgisches Handstück nach einem der Ansprüche 11 bis 13,
**dadurch gekennzeichnet,**
**dass** dieses in der Griffhülse (110) einen Antriebsmotor oder eine zur Kopplung mit einem externen Antriebsmotor vorgesehene Welle (115) aufweist, wobei ferner in der Griffhülse (110) ein Antriebsstrang (116) gelagert ist zur Übermittlung einer Rotationsbewegung auf das an dem Kopfstück (50) gehaltene Werkzeug (120).

## Claims

1. Headpiece (50) for a dental or surgical handpiece (100),
wherein the headpiece (50) serves to releasably receive a dental tool (120),
wherein a clamping mechanism (20) for releasably holding the tool (120) is formed in the headpiece (50), which has a pushbutton (10) for actuating the clamping mechanism (20),
**characterized**
**in that** the pushbutton (10) consists of a thermally insulating, electrically non-conductive plastic or ceramic material in which a plate-shaped recess (11) is formed, an RFID transponder (5) provided for identifying the handpiece (100) being arranged in said plate-shaped recess.

2. Headpiece for a dental or surgical handpiece according to Claim 1,
**characterized**
**in that** the RFID transponder (5) arranged in the recess (11) is potted using a potting compound (15).

3. Headpiece for a dental or surgical handpiece according to Claim 2,
**characterized**
**in that** the RFID transponder (5) is at a distance from a base surface (12) of the plate-shaped recess (11), wherein the region between the RFID transponder (5) and the base surface (12) is filled by the potting compound (15) .

4. Headpiece for a dental or surgical handpiece according to Claim 2 or 3,
**characterized**
**in that** the potting compound (15) filling the plate-shaped recess (11) and surrounding the RFID transponder (5) forms a region (16) that is raised in relation to the surrounding surface region (13) of the pushbutton (10), wherein the recess (11) and the raised region (16) preferably have an approximately circular outer contour.

5. Headpiece for a dental or surgical handpiece according to Claim 4,
**characterized**
**in that** the maximum projection of the potting compound (15) in relation to the surrounding surface region (13) of the pushbutton (10) is less than 2 mm, preferably 1 mm to 2 mm.

6. Headpiece for a dental or surgical handpiece according to Claim 5,
**characterized**
**in that** the projecting potting compound (15) substantially consistently continues the surrounding surface region (13) of the pushbutton (10).

7. Headpiece for a dental or surgical handpiece according to one of the preceding claims,
**characterized**
**in that** the pushbutton (10) is arranged on a top side of the headpiece (50) situated opposite the tool (120), wherein the plate-shaped recess (11) is preferably formed substantially centrally on the pushbutton (10).

8. Headpiece for a dental or surgical handpiece according to one of the preceding claims,
**characterized**
**in that** the plastic is polyether ether ketone (PEEK).

9. Headpiece for a dental or surgical handpiece according to one of the preceding claims,
**characterized**
**in that** the potting compound (15) contains resin and/or silicone.

10. Headpiece for a dental or surgical handpiece according to one of the preceding claims,
**characterized**
**in that** the clamping mechanism (20) has a collet (25) holding a shaft of the tool (120).

11. Dental or surgical handpiece comprising
an elongate grip sleeve (110) and
a headpiece (50) according to one of the preceding claims arranged at the front end of the grip sleeve (110).

12. Dental or surgical handpiece according to Claim 11,
**characterized**
**in that** the grip sleeve (110) and the headpiece (50) consist of metal.

13. Dental or surgical handpiece according to Claim 11 or 12,
**characterized**
**in that** the grip sleeve (110) is of angled design.

14. Dental or surgical handpiece according to one of Claims 11 to 13,
**characterized**
**in that** it has a drive motor or a shaft (115) provided for coupling to an external drive motor in the grip sleeve (110), wherein a drive train (116) is further mounted in the grip sleeve (110) for the purpose of transmitting a rotational movement to the tool (120) held on the headpiece (50).

## Revendications

1. Pièce de tête (50) destinée à une pièce à main (100) à usage dentaire ou chirurgical,
la pièce de tête (50) servant à recevoir un outil de dentisterie (120) de manière amovible,
un mécanisme de serrage (20) destiné à maintenir l'outil (120) de manière amovible étant prévu dans la pièce de tête (50), lequel mécanisme de serrage comporte un bouton poussoir (10) destiné à actionner le mécanisme de serrage (20),
**caractérisée en ce que**
le bouton poussoir (10) est en une matière synthétique ou céramique thermo-isolante et électriquement non conductrice dans laquelle est ménagé un creux (11) en forme de plaque dans lequel est disposé un transpondeur RFID (5) prévu pour l'identification de la pièce à main (100) .

2. Pièce de tête destinée à une pièce à main à usage dentaire ou chirurgical selon la revendication 1,
**caractérisée en ce que**
le transpondeur RFID (5) disposé dans le creux (11) est enrobé avec une matière d'enrobage (15).

3. Pièce de tête destinée à une pièce à main à usage dentaire ou chirurgical selon la revendication 2,
**caractérisée en ce que**
le transpondeur RFID (5) est à distance d'une surface de fond (12) du creux (11) en forme de plaque, la zone située entre le transpondeur RFID (5) et la zone de fond (12) étant remplie avec la matière d'enrobage (15) .

4. Pièce de tête destinée à une pièce à main à usage dentaire ou chirurgical selon la revendication 2 ou 3,
**caractérisée en ce que**
la matière d'enrobage (15) qui remplit le creux (11) en forme de plaque et qui entoure le transpondeur RFID (5) forme une zone (16) qui est surélevée par rapport à la zone de surface environnante (13) du bouton-poussoir (10), le creux (11) et la zone surélevée (16) comportant de préférence un contour extérieur à peu près circulaire.

5. Pièce de tête destinée à une pièce à main à usage dentaire ou chirurgical selon la revendication 4,
**caractérisée en ce que**
la projection maximale de la matière d'enrobage (15) par rapport à la zone de surface environnante (13) du bouton-poussoir (10) est inférieure à 2 mm, de préférence va de 1 mm à 2 mm.

6. Pièce de tête destinée à une pièce à main à usage dentaire ou chirurgical selon la revendication 5,
**caractérisée en ce que**
la matière d'enrobage (15) en projection prolonge la zone de surface environnante (13) du bouton-poussoir (10) de manière sensiblement continue.

7. Pièce de tête destinée à une pièce à main à usage dentaire ou chirurgical selon l'une des revendications précédentes,
**caractérisée en ce que**
le bouton-poussoir (10) est disposé sur un côté supérieur de la pièce de tête (50) qui est opposé à l'outil (120), le creux (11) en forme de plaque étant formé sur le bouton-poussoir (10) de préférence sensiblement au milieu.

8. Pièce de tête destinée à une pièce à main à usage dentaire ou chirurgical selon l'une des revendications précédentes,
**caractérisée en ce que**
la matière synthétique est du polyétheréthercétone (PEEK).

9. Pièce de tête destinée à une pièce à main à usage dentaire ou chirurgical selon l'une des revendications précédentes,
**caractérisée en ce que**
la matière d'enrobage (15) contient de la résine et/ou du silicone.

10. Pièce de tête destinée à une pièce à main à usage dentaire ou chirurgical selon l'une des revendications précédentes,
**caractérisée en ce que**
le mécanisme de serrage (20) comporte une pince de serrage (25) maintenant une tige de l'outil (120).

11. Pièce à main à usage dentaire ou chirurgical comprenant
un manchon de préhension allongé (110) et
une pièce de tête (50) selon l'une des revendications précédentes qui est disposée à l'extrémité avant du manchon de préhension (110).

12. Pièce à main à usage dentaire ou chirurgical selon la revendication 11,
**caractérisée en ce que**
le manchon de préhension (110) et la pièce de tête (50) sont en métal.

13. Pièce à main à usage dentaire ou chirurgical selon la revendication 11 ou 12,
**caractérisée en ce que**
le manchon de préhension (110) est coudé.

14. Pièce à main à usage dentaire ou chirurgical selon l'une des revendications 11 à 13,
**caractérisée en ce que**
celle-ci comporte dans le manchon de préhension (110) un moteur d'entraînement ou un arbre (115) prévu pour l'accouplement à un moteur d'entraînement extérieur, une chaîne cinématique (116) étant en outre montée dans le manchon de préhension (110) afin de transmettre un mouvement de rotation à l'outil (120) maintenu sur la pièce de tête (50).
